# EUROPEAN PATENT SPECIFICATION

(11) **EP 2 709 517 B1**
(45) Date of publication and mention of the grant of the patent: **11.01.2017**
(21) Application number: 12785788.6
(22) Date of filing: 17.05.2012
(51) Int. Cl.: A61B 5/0215, A61B 5/20, A61B 5/00, A61B 18/00, A61B 18/14

(54) **APPARATUS FOR ASSESSING TRANSVASCULAR DENERVATION**
VORRICHTUNG ZUR BEURTEILUNG EINER TRANSVASKULÄREN DENERVIERUNG
APPAREIL D'ÉVALUATION DE LA DÉNERVATION TRANSVASCULAIRE

(30) Priority: 18.05.2011 US 201113110041; 18.05.2011 US 201113110032; 06.06.2011 US 201113153838; 06.06.2011 US 201113153810
(43) Date of publication of application: 26.03.2014
(73) Proprietor: St. Jude Medical, Inc., St. Paul, MN 55117-9913 (US)
(72) Inventor: NG, Kok-Hwee, Irvine California 92620 (US)
(74) Representative: Smaggasgale, Gillian Helen
(86) International application number: PCT/US2012/038247
(87) International publication number: WO 2012/158864

(56) References cited:
- US-A- 5 312 339
- US-A1- 2005 288 730
- US-A1- 2006 030 845
- US-A1- 2007 060 921
- US-A1- 2010 076 426
- US-A1- 2010 087 782
- US-A1- 2010 113 939
- US-A1- 2010 222 851
- US-A1- 2010 292 602
- US-A1- 2010 317 921
- US-A1- 2011 040 232
- US-B1- 6 192 275
- US-B2- 7 749 220

## Description

### BACKGROUND OF THE INVENTION

The present invention relates generally to catheter devices, and more specifically to catheter devices for assessing transvascular denervation.

Renal denervation is a method whereby amplified sympathetic activities are suppressed to treat hypertension or other cardiovascular disorders and chronic renal diseases. The objective of renal denervation is to neutralize the effect of renal sympathetic system which is involved in arterial hypertension. The following describes some examples of renal denervation devices. U.S. Patent No. 7,653,438 discloses renal neuromodulation using a pulsed electric field to effectuate electroporation or electrofusion. It describes percutaneous intravascular delivery of pulsed electric fields to achieve renal neuromodulation. U.S. Patent Application Publication No. 2010/0268307 discloses intravascularly induced neuromodulation using a pulsed electric field to effectuate irreversible electroporation or electrofusion, necrosis, and/or inducement of apoptosis, alteration of gene expression, changes in cytokine upregulation, etc., in target neural fibers. It mentions the use of the technique to modulate a neural fiber that contributes to renal function. International Patent Publication No. WO2008/092246 discloses transvascular nerve stimulation through the walls of blood vessels. It uses electrodes supported on an electrically insulating backing sheet and a structure for holding the backing sheet against the inner wall of the blood vessel.

Document US 2005/288730 A1 discloses a device for renal neuromodulation comprising a deployable structure coupled to a catheter. The deployable structure carries electrodes.

Catheters are flexible, tubular devices that are widely used by physicians performing medical procedures to gain access into interior regions of the body. A catheter device can be used for ablating renal sympathetic nerves in therapeutic renal sympathetic denervation to achieve reductions of blood pressure in patients suffering from renal sympathetic hyperactivity associated with hypertension and its progression. See, e.g., Henry Krum et al., Catheter-Based Renal Sympathetic Denervation for Resistant Hypertension: A Multicentre Safety and Proof-of-Principle Cohort Study, published online March 30, 2009 at www.thelancet.com.

Renal arteries, like all major blood vessels, are innervated by perivascular sympathetic nerves that traverse the length of the arteries. The perivascular nerves consist of a network of axons, terminals, and varicosities, which are distributed mostly in the medial-adventitial and adventitial layers of the arterial wall. The medial and adventitial layers of the renal artery consist mostly of vascular smooth muscle cells (VSMCs), while the intimal layer is made up of endothelial cells (ECs). A small percentage of the VSMCs in the adventitia of the renal artery have a direct coupling to the varicosities because of their proximity to each other. When the renal sympathetic nerve is stimulated and an action potential travels along the axon, all varicosities along the axon are depolarized and neurotransmitters are released into the junctions of directly-coupled VSMCs. This event causes the opening of specific ionic and second messenger molecule channels of the VSMCs and results in their depolarization.

The depolarization described above would normally be confined to the first layer of the VSMCs that are directly coupled to the varicosities because extracellular diffusion of neurotransmitters is characterized by a small space and time constant. Therefore in theory any recording of excitatory junction potentials (EJPs) should be done at the directly coupled VSMCs. However, numerous studies of vasomotion of arteries have led to the understanding that additional VSMCs are recruited in generating a syncytial response to neural stimulation. Thus gap junctions have been identified amongst VSMCs and ECs, and between VSMCs and ECs. These gap junctions form the conduit for intercellular communication and facilitate neurotransmitter diffusion over a larger space constant than can be accounted for by extracellular diffusion alone. Intercellular diffusion is dependent on gap junction permeability and decays as it crosses each junction. Recording of EJPs have been shown to take place within a few millimeters from perivascular nerves.

EJP recordings are commonly performed using intracellular techniques. The recording electrodes are typically made of glass micropipettes that impale a single cell to provide an isolated signal path to an amplifier. In other techniques, suction is applied to provide a high impedance seal between the glass micropipette and cell membrane without having to penetrate the cell. The high impedance seal isolates signal conduction between the cell membrane and the electrode from environmental noise. Extracellular recording of junction potentials are possible but more challenging because of the much smaller potential amplitude at the surface of the cell and the electronics requirements for noise reduction. Depending on the size of the recording electrode, extracellular recording may record from a single cell or an ensemble of cells. The latter is analogous to field potential recording from multi-neurons in the brain.

In certain animal arteries such as rat tail arteries and mesenteric arteries, recording of spontaneous EJPs (SEJPs) have been reported. These SEJPs occur asynchronously following normal sympathetic activities of the animal physiology. On average the recording of these asynchronous SEJPs will cancel out within a field potential recording technique, regardless of the fidelity of the recording electronics. On the other hand, when perivascular nerves are stimulated under external control, EJPs occur synchronously with the stimulus source. Occasionally when a sufficient number of perivascular nerve fibers are activated at the same time, the EJPs become suprathreshold and muscle action potentials are initiated thus further enhancing evoked responses to be detected in the recording. Common techniques for electrical perivascular nerve stimulation include both nerve trunk stimulation and transmural (field) stimulation. Both techniques require direct electrode access to the adventitial area of the artery. With transmural stimulation, the stimulus strengths are designed to be supramaximal to activate the perivascular nerves without directly stimulating the VSMCs.

Transvascular technique of electrical stimulation of perivascular nerves is not as widely reported. One possible explanation is the stimulus strength required to activate the perivascular nerves will most likely also stimulate the VSMCs directly (this is because the electrical current density from the electrode attenuates as a function of distance and the VSMCs are closer to the stimulating electrodes than the perivascular nerves), thus making it difficult to analyze neurally evoked responses independently. Most electrical stimulus consists of square pulses and by using shorter pulse widths it may be possible to selectively activate the perivascular nerves only. However, a better method is to make use of the anisotropy of VSMCs and the finite space constants of EJPs to differentiate neurally evoked responses from field evoked responses, so that perivascular nerve integrity can be independently assessed. Thus in a preferred arrangement, the stimulating electrodes and recording electrodes should be separated by a distance greater than the EJP space constant in the longitudinal direction of the vessel. In addition, the stimulating electrodes should be located proximally and recording electrodes distally to selectively record orthodromic nerve traffic. In this way, nerve action potentials or muscle action potentials evoked by orthromic stimulation of the axons are enhanced, while EJPs evoked in the vicinity of the stimulating electrodes are excluded.

### BRIEF SUMMARY OF THE INVENTION

Prior denervation devices do not provide any means of predicting the long term outcome of the renal denervation therapies. Nor do they provide a marker to assess the completeness of the therapeutic procedure. In the case where denervation is carried out by delivering continuous RF energy through the vascular wall, the typical target procedural parameters are impedance, elapsed time, temperature or power, or a combination of all the above. The correlation of these parameters with the extent of denervation has never been shown, and may not be tenable given the heterogeneous nature of vascular innervation structures. There is therefore a need to provide a more direct technique of measuring residual neural activities following denervation in order to assess the completeness of such procedures.

Exemplary embodiments of the invention provide catheter devices for assessing transvascular denervation. The patency of the innervation of vessels can be assessed directly through electrophysiological techniques. Specifically, appropriately positioned sensors are deployed in direct contact with the vessel luminal wall to record evoked responses from external stimulus. Evoked biopotentials from the vascular wall can be recorded. Mechanical responses (vasoconstriction or vasodilation) associated with these neural events can also be monitored. By comparing the pre-treatment recording with post-treatment recording, a DeNervation Assessment index (DNAi) can be derived.

In accordance with an aspect of the present disclosure, a catheter apparatus for assessing denervation comprises: an elongated catheter body having a proximal end and a distal end, a longitudinal axis extending in a longitudinal direction between the distal end and the proximal end; a deployable structure coupled to the catheter body, the deployable structure being deployable outwardly from the longitudinal axis and contractible inwardly toward the longitudinal axis of the catheter body; one or more ablation elements disposed on the deployable structure to move outwardly and inwardly with the deployable structure, the one or more ablation elements being powered to apply ablation energy to a vessel of a patient; one or more stimulation elements spaced from each other and disposed on the deployable structure to move outwardly and inwardly with the deployable structure, the one or more stimulation elements being powered to supply nerve stimulating signals to the vessel; and one or more recording elements spaced from each other and from the one or more stimulation elements, the one or more recording elements being disposed on the deployable structure to move outwardly and inwardly with the deployable structure, the one or more recording elements configured to record response of the vessel to the nerve stimulating signals.

In some embodiments, the one or more stimulation elements are proximal relative to the one or more recording elements. A most distal ablation element of the one or more ablation elements is no more distal than at least one of the stimulation elements and a most proximal ablation element of the one or more ablation elements is no more proximal than at least one of the one or more recording elements. At least one of the ablation elements is also a stimulation element or a recording element. Some of the one or more recording elements are spaced from one of the one or more stimulation elements by one of a longitudinal spacing, a lateral spacing, or a combined longitudinal and lateral spacing. The one or more recording elements are configured to record one or more of evoked electrical responses or mechanical responses of the vessel in response to the nerve stimulating signals.

In specific embodiments, the deployable structure comprises a plurality of longitudinal spines, each of the spines having a proximal end connected to the catheter body and a distal end connected to the catheter body. Each spine includes an elbow having at least one discontinuity in stiffness at an intermediate position between the distal end and the proximal end thereof. The one or more ablation elements, the one or more stimulation elements, and the one or more recording elements are disposed on the spines. The deployable structure is contractible to a contracted arrangement to fit within a lumen of the elongated catheter body and is deployable to a deployed arrangement with the elbows of the spines bending outwardly relative to the proximal and distal ends of the spines, the elbows of the spines moving radially outwardly from the contracted arrangement to the deployed arrangement. The catheter apparatus further comprises a balloon disposed inside the deployable structure, the spines being disposed radially outwardly relative to the balloon, the balloon inflating to move the spines radially outwardly in the deployed arrangement and deflating in the contracted arrangement. One or more contact sensors are disposed on a surface of the balloon for measuring force or pressure.

In some embodiments, the deployable structure comprises a balloon made of an electrically insulative material, the balloon inflating to move radially outwardly relative to the catheter body in a deployed arrangement and deflating in an undeployed arrangement. The one or more ablation elements, one or more stimulation elements, and one or more recording elements are disposed on a surface of the balloon. An intraluminal pressure sensor is to be introduced and deployed inside the balloon for pressure monitoring.

In specific embodiments, the deployable structure comprises a deployable sleeve made of an electrically insulative material and a hollow tubing disposed inside the deployable sleeve. The hollow tubing includes a plurality of holes for fluid to pass therethrough to push the deployable sleeve radially outwardly relative to the catheter body in a deployed arrangement. The one or more ablation elements, the one or more stimulation elements, and the one or more recording elements are disposed on a surface of the deployable sleeve. The catheter apparatus further comprises a plurality of draw strings extending from inside the hollow tubing through the holes to the deployable sleeve to draw the deployable sleeve radially inwardly relative to the catheter body in an undeployed arrangement. The deployable structure is contractible to a contracted arrangement and is deployable to a deployed arrangement. The deployable structure includes an anti-occlusion feature to permit fluid flow in the vessel between a proximal end and a distal end of the deployable structure in the deployed arrangement.

In accordance with another aspect of the disclosure a catheter apparatus for assessing denervation comprises: an elongated catheter body having a proximal end and a distal end, a longitudinal axis extending in a longitudinal direction between the distal end and the proximal end; a structure coupled to the catheter body; one or more ablation elements disposed on the structure and being powered to apply ablation energy to a vessel of a patient; one or more stimulation elements spaced from each other and disposed on the structure, the one or more stimulation elements being powered to supply nerve stimulating signals to the vessel; one or more recording elements spaced from each other and from the one or more stimulation elements, the one or more recording elements being disposed on the structure and configured to record response of the vessel to the nerve stimulating signals; and a mechanism to deploy the structure outwardly from the longitudinal axis of the catheter body to move the ablation elements, the one or more stimulation elements, and the one or more recording elements outwardly to a deployed arrangement, and to contract the structure inwardly toward the longitudinal axis of the catheter body to move the ablation elements, the one or more stimulation elements, and the one or more recording elements inwardly to a contracted arrangement.

In accordance with another aspect of this disclosure, a method of assessing denervation comprises: introducing intravascularly a catheter to a vessel of a patient, the catheter including one or more recording elements; performing a baseline recording of responses by supplying nerve stimulation to the vessel and recording responses of the vessel to the nerve stimulation; denervating at least some tissue proximate the vessel after performing the baseline recording; performing a post-denervation recording of responses, after the denervating, by supplying nerve stimulation to the vessel and recording responses of the vessel to the nerve stimulation; and assessing denervation of the vessel based on a comparison of the responses of the baseline recording and the responses of the post-denervation recording.

In some embodiments, the catheter is not repositioned during performing the baseline recording, denervating, and performing the post-denervation recording. The nerve stimulation comprises one of electrical stimulation or pharmacological stimulation. The responses include one or more of electrical response or mechanical response. Assessing denervation of the vessel comprises: computing a baseline parameter from the responses of the baseline recording; computing a post-denervation parameter from the responses of the post-denervation recording; and computing a degree of denervation as a ratio of the post-denervation parameter and the baseline parameter. Denervation is achieved when the computed ratio falls within a preset range. The baseline parameter is computed based on a baseline maximum signal amplitude of one or more evoked responses generated in response to the nerve stimulation and recorded by the one or more recording elements before the denervating. The post-denervation is computed based on a post-denervation maximum signal amplitude of one or more evoked responses generated in response to the same nerve stimulation and recorded by the one or more recording elements after the denervating. The one or more evoked responses include one or more of evoked electrical response or evoked mechanical response.

In specific embodiments, the baseline parameter is computed based on a baseline area under a plot of one or more evoked responses generated in response to the nerve stimulation and recorded by the one or more recording elements before the denervating. The post-denervation is computed based on a post-denervation area under a plot of one or more evoked responses generated in response to the same nerve stimulation and recorded by the one or more recording elements after the denervating. The method further comprises, if denervation of the vessel is not achieved, repeating the steps of denervating, performing a post-denervation recording of responses, and assessing denervation of the vessel until denervation of the vessel is achieved. The repeating until denervation of the vessel is achieved is performed in real time. The catheter is not repositioned during the repeating. Repeating the steps of denervating, performing a post-denervation recording of responses, and assessing denervation of the vessel comprises adjusting a level of denervation for denervating at least some tissue proximate the vessel based on result of assessing denervation of the vessel. The repeating including the adjusting until denervation of the vessel is achieved is performed in real time. In another embodiment, ablation cycles are interleaved with neural assessment cycles throughout the entire therapeutic procedure, with pre-determined duty cycles assigned between ablation and neural assessment (interleaved ablation and assessment mode). In this way, neural assessment can begin as soon ablation begins, instead of deferring until a pre-set dose of ablation energy has been delivered. The interleave frequency is preferably about 10 to 50 Hz, and the duty cycle for ablation is preferably about 50 to 90%.

In some embodiments, recording responses of the vessel to the nerve stimulation comprises recording one or more of evoked electrical responses or mechanical responses of the vessel in response to the nerve stimulation. The vessel is denervated using one or more ablation elements disposed on the catheter. The catheter is not repositioned during performing the baseline recording, denervating, and performing the post-denervation recording. The catheter includes one or more stimulation elements to supply nerve stimulating signals as the nerve stimulation.

In specific embodiments, the method further comprises deploying a deployable structure to enhance contact between the one or more stimulation elements and the vessel and between the one or more recording elements and the vessel. The method further comprises providing electrical insulation between the one or more stimulation elements and the one or more recording elements to facilitate signal conduction between the one or more stimulation elements and tissue proximate the vessel and to facilitate signal conduction between the one or more recording elements and tissue proximate the vessel. The method further comprises deploying a deployable structure to enhance contact between the one or more recording elements and the vessel.

These and other features and advantages of the present invention will become apparent to those of ordinary skill in the art in view of the following detailed description of the specific embodiments.

### BRIEF DESCRIPTION OF THE DRAWINGS

FIG. 1 is an elevational view of a catheter device with a deployable basket for assessing transvascular denervation according to an embodiment of the present invention. FIG.1 is provided for information and does not form part of the invention
FIG. 2 is an elevational view of a catheter device with a balloonaugmented deployable basket according to another embodiment of the present invention. FIG.2 is provided for information and does not form part of the invention
FIG. 3 is an elevational view of a catheter device with a deployable balloon according to another embodiment of the present invention. FIG.3 is provided for information and does not form part of the invention
FIG. 4 is a perspective view of a catheter device with a deployable sleeve according to another embodiment of the present invention.
FIG. 5 shows different configurations of the stimulating and recording electrodes of the deployable sleeve of FIG. 4.
FIG. 6 is a perspective view of a partially cut-out section of the catheter device of FIG. 4.
FIG. 7 is a schematic view illustrating an apparatus to provide an intraluminal pressure sensor for use with the catheter device.
FIG. 8 is a perspective view of a catheter device with a deployable sleeve having an anti-occlusion feature according to another embodiment of the present invention.
FIGS. 8A and 8B are elevational views of a catheter device with a deployable sleeve having an anti-occlusion feature according to another embodiment of the present invention.
FIG. 9 shows an example of a flow diagram illustrating a method for assessing transvascular denervation.
FIG. 10 shows an example of a plot of evoked responses and stimulus artifacts recorded by the recording elements.

### DETAILED DESCRIPTION OF THE INVENTION

In the following detailed description of the invention, reference is made to the accompanying drawings which form a part of the disclosure, and in which are shown by way of illustration, and not of limitation, exemplary embodiments by which the invention may be practiced. In the drawings, like numerals describe substantially similar components throughout the several views. Further, it should be noted that while the detailed description provides various exemplary embodiments, as described below and as illustrated in the drawings, the present invention is not limited to the embodiments described and illustrated herein, but can extend to other embodiments, as would be known or as would become known to those skilled in the art. The appearance of phrases "one embodiment", "this embodiment", or "these embodiments" means that a particular feature, structure, or characteristic described in connection with the embodiment is included in at least one embodiment of the invention, and the appearances of these phrases in various places in the specification are not necessarily all referring to the same embodiment. Additionally, in the following detailed description, numerous specific details are set forth in order to provide a thorough understanding of the present invention. However, it will be apparent to one of ordinary skill in the art that these specific details may not all be needed to practice the present invention. In other circumstances, well-known structures, materials, circuits, processes and interfaces have not been described in detail, and/or may be illustrated in block diagram form, so as to not unnecessarily obscure the present invention.

In the following description, relative orientation and placement terminology, such as the terms horizontal, vertical, left, right, top and bottom, is used. It will be appreciated that these terms refer to relative directions and placement in a two dimensional layout with respect to a given orientation of the layout. For a different orientation of the layout, different relative orientation and placement terms may be used to describe the same objects or operations.

Exemplary embodiments of the invention, as will be described in greater detail below, provide apparatuses and methods for assessing transvascular denervation.

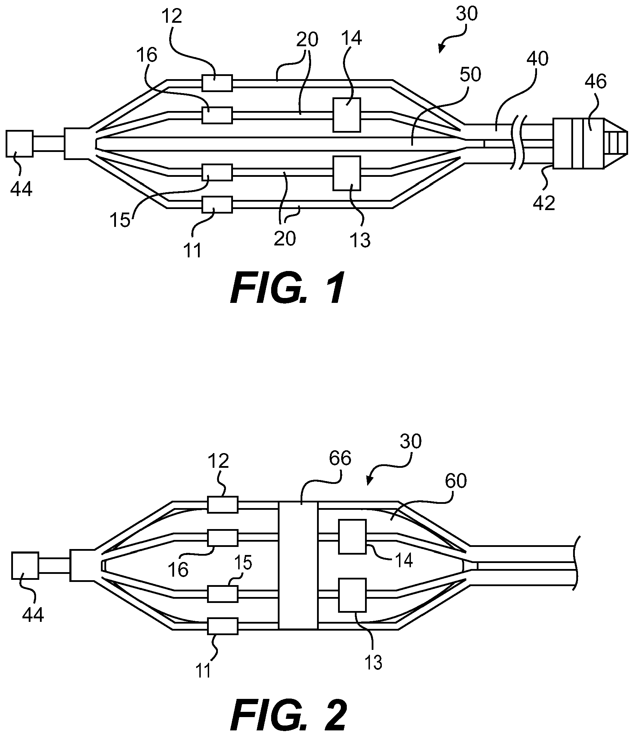

FIG. 1 is an elevational view of a catheter device with a deployable basket for assessing transvascular denervation . The device integrates neural assessment elements with denervation elements for renal denervation or the like. The denervation elements are configured as ablation electrodes. Alternatively, the ablation/denervation element may employ other mechanisms or other types of energy (e.g., laser, high intensity focused ultrasound (HIFU), cryoablation, mechanical) to sever or interrupt conduction of the nerve fibers. FIG. 1 shows ablation electrodes 11, 12, 13, 14 and additional electrodes 15, 16 which are disposed on spines 20 in a deployable structure in a basket configuration 30 which is coupled to a catheter body 40. The catheter body 40 has a proximal end 42, a distal end 44, and a longitudinal axis extending in a longitudinal direction between the distal end 44 and the proximal end 42. An external catheter handle 46 is provided at the proximal end. The spines 20 are electrically nonconductive.

The deployable basket 30 is coupled to the distal portion of the catheter body 40, and is deployable or expandable outwardly from the longitudinal axis and contractible inwardly toward the longitudinal axis of the catheter body 40. The basket 30 has a plurality of longitudinal spines 20 having distal ends and proximal ends that are attached to the catheter body 40. Each spine 20 includes an elbow having at least one discontinuity in stiffness at an intermediate position between the distal end and the proximal end thereof to allow the spine to expand/deploy and collapse/contract. Different mechanisms can be used to cause the expansion and collapse of the spines 20. In FIG. 1, a pull wire 50 extending along the longitudinal axis of the catheter body 40 is used to pull the distal end of the basket 30 in the proximal direction to deploy the basket 30. The basket 30 is in a deployed configuration in FIG. 1. To return the basket 30 to a contracted/undeployed configuration with the spines 20 extending generally longitudinally in the longitudinal direction, the spines 20 can be resiliently biased toward the contracted configuration and the pulling force on the pull wire 50 can be released to allow the basket 30 to contract. Alternatively, the pull wire 50 may be configured to apply a push force to push the distal end of the basket 30 in the distal direction to contract the basket 30.

The basket 30 is contractible to a contracted arrangement to fit within a lumen of the elongated catheter body 40 and is deployable to a deployed arrangement with the elbows of the spines bending outwardly relative to the proximal and distal ends of the spines 20, the elbows of the spines moving radially outwardly from the contracted arrangement to the deployed arrangement. An example of a similar deployable basket is found in US 2010/0076426 entitled Basket Catheter Having Multiple Electrodes, which is incorporated herein by reference in its entirety.

The pull wire 50 extends through a lumen of the catheter body 40. The lumen can also accommodate lines for supplying power to ablation electrodes, signals lines to stimulation electrodes and recording electrodes, fluid lines, and the like.

The electrodes 11, 12, 13, 14, 15, 16 are disposed on the deployable basket 30 to move outwardly and inwardly with the basket 30. The ablation electrodes 11, 12, 13, 14 are powered to apply ablation energy to a vessel of a patient such as a renal artery. Any of the ablation electrodes can also be stimulation or recording electrodes. Stimulation electrodes are powered to supply nerve stimulating signals to the vessel. The nerve stimulating signals are typically about 1 Hz to about 16 Hz and are designed to cause nerve excitation and evoke neurotransmitter release from perivascular varicosities. The stimulation thresholds will likely be much smaller than those for neuromodulation as taught in the art, since the intent is not to irreversibly electroporate the nerves. Recording electrodes are configured to record response of the vessel to the nerve stimulating signals.

The recording electrodes may be configured to record one or more of evoked electrical responses or mechanical responses of the vessel in response to the nerve stimulating signals. The stimulation electrodes are spaced from each other, the recording electrodes are spaced from each other, and the stimulation electrodes are spaced from the recording electrodes. As seen in FIG. 1, the spacing between the electrodes can be longitudinal, lateral/circumferential, or a combination of the two. Several stimulation and recording configurations are possible in FIG. 1. A first example includes stimulation electrodes 13, 15 and recording electrodes 14, 16 and 11, 12. A second example includes stimulation electrodes 14, 16 and recording electrodes 13, 15, and 11, 12. A third example includes stimulation electrodes 11, 12 and recording electrodes 13, 15 and 14, 16. In some preferred embodiments, at least some of the stimulation electrodes are proximal relative to at least some of the recording electrodes. The stimulation electrodes 14, 16 are separated from the recording electrodes 11, 12, 13, 15 by at least a minimum distance, which is preferably approximately equal to the space constant of excitatory junction potentials (EJPs).

In FIG. 1, ablation is performed with two distal electrodes (11, 12) and two proximal electrodes (13, 14). Neural assessment is performed with a combination of the four ablation electrodes (11, 12, 13, 14) which also serve as stimulation or recording electrodes and two additional sensor electrodes (15, 16). In neural assessment, stimulating electrodes are typically parallel to the long axis of the vessel, while recording electrodes are made up of both transverse pairs and parallel pairs to the long axis of the vessel. Stimulation and recording typically involve separate sets of electrodes, but can be configured for simultaneous stimulation and recording with appropriate electronics circuits. Stimulation and recording can be performed using bipolar electrodes as shown in FIG. 1. Alternatively, monopolar and multipolar techniques can be used. For monopolar stimulation or recording, only one active electrode is required and an indifferent electrode is provided on the patient's tissue. Ablation is typically performed monopolarly, but bipolar or multipolar ablations are also possible with a different electrode arrangement. In some preferred embodiments, the most distal ablation electrode (11 or 12) is no more distal than at least one of the stimulation electrodes and the most proximal ablation electrode (13 or 14) is no more proximal than at least one of the recording electrodes.

FIG. 2 is an elevational view of a catheter device with a balloon-augmented deployable basket . This is similar to that of FIG. 1 with the addition of an elongated balloon 60 inside the basket 30 to replace the pullwire expansion mechanism 50. It inflates to expand the basket 30 outwardly in the deployed arrangement and deflates in the contracted/undeployed arrangement. This enhances tissue and electrode contact for both ablation and neural assessment. The balloon-augmented catheter is first deflated and positioned at the target renal artery site. Then the balloon 60 is inflated with saline or the like to a preset volume for neural assessment and ablation. The balloon 60 is preferably made of an electrically insulative material, which facilitates signal conduction between the stimulation electrodes and the tissue proximate the vessel and to facilitate signal conduction between the recording electrodes and the tissue proximate the vessel. The electrically insulative balloon 60 preferably permits the least resistive path for signal conduction between the stimulation electrodes and the tissue and between the recording electrodes and the tissue.

The contact force or pressure between the vessel wall and the balloon 60 is transmitted to the fluid inside the balloon 60 and measured proximally at the external catheter handle 46 via suitable instruments; alternatively, additional electromechanical contact force/pressure sensors 66 can be incorporated on the surface of the balloon 60 to record changes in the luminal wall force/pressure. FIG. 2 shows a circumferential band of contact force/pressure sensors 66, but other sensor configurations are possible. The method of mechanical sensing here is advantageous since it is less susceptible to interference with the source of stimulus which is of a different modality (electrical). The balloon 60 may be made of a variety of materials including, for example, polyurethane or nylon.

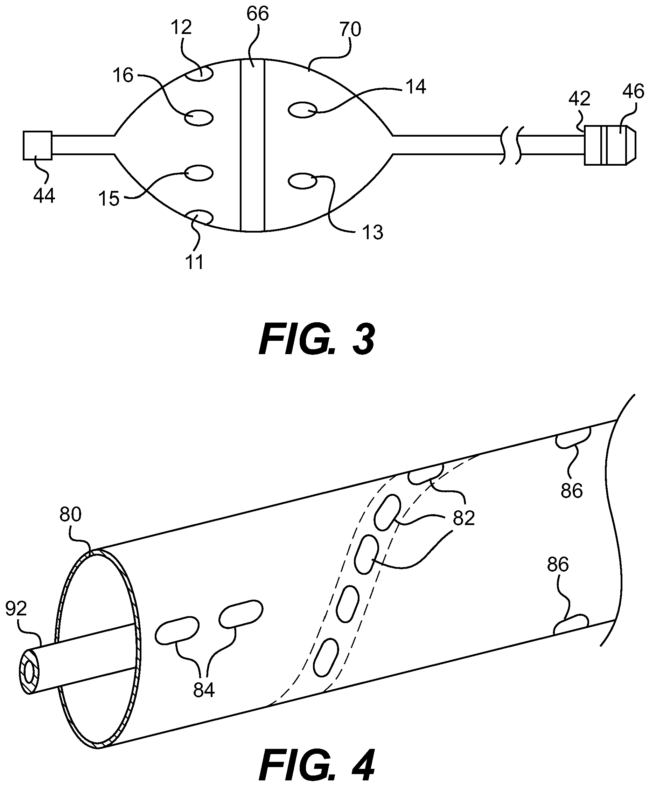

FIG. 3 is an elevational view of a catheter device with a deployable balloon This is similar to that of FIG. 2 but eliminates the basket and employs a balloon 70 with integrated electrodes. The electrodes may be embedded with the balloon material or printed on through a thin film deposition process. For simplicity, FIG. 3 shows a similar set of electrodes 11, 12, 13, 14, 15, 16 and force/pressure sensors 66 as those in FIGS. 1 and 2. The balloon 70 may have some or all of the same features and characteristics as the balloon 60 of FIG. 2 as described above.

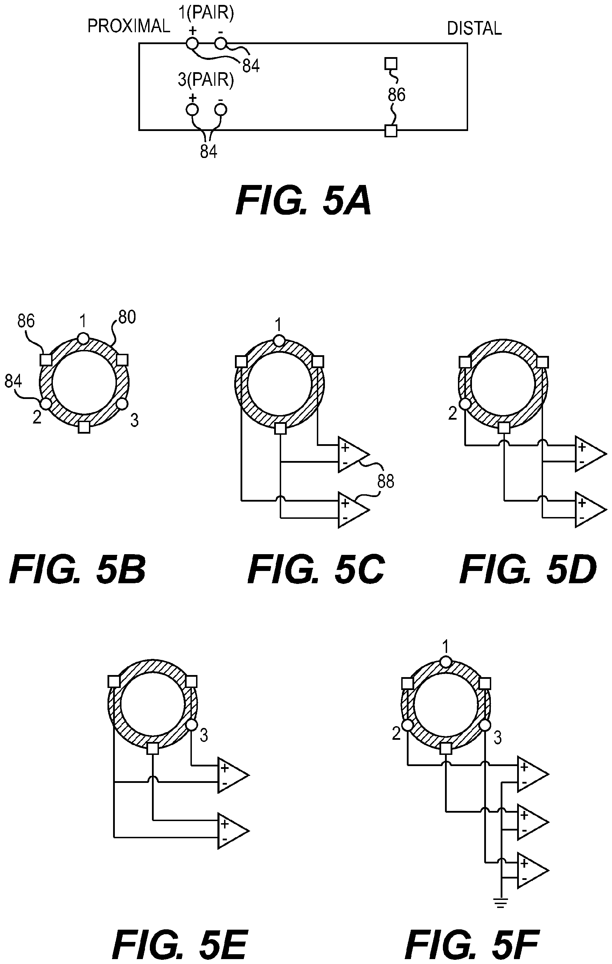

FIG. 4 is a perspective view of a catheter device with a deployable sleeve according to an embodiment of the present invention. This embodiment makes use of a deployable sleeve 80 instead of a balloon to insulate the electrodes from the blood in the vessel. The sleeve 80 is preferably made of an electrically insulating material. Various arrangements of electrodes for ablation and assessing denervation can be utilized. As an example, a spiral strip (not shown) of ablation electrode can be attached to the outside of the sleeve 80 and along the long axis of the sleeve 80. Alternatively, the single strip may be divided into multiple ablation electrodes 82 connected in series to form a spiral arrangement along the outside of the sleeve 80. This is the arrangement, for example, when monopolar ablation is desired and output power is divided equally across all ablation electrodes. Alternatively, ablation can be performed preferentially by bipolar means. In this case, pairs of electrodes can be grouped to create bipolar lesions that are larger and more contiguous than if monopolar lesions were created. A plurality of bipolar sets of stimulation electrodes 84 (FIGS. 4 and 5 show three pairs each being parallel to the long axis of the sleeve 80) and a plurality of recording electrodes 86 (FIGS. 4 and 5 show three) are attached to each side of the ablation electrodes 82 respectively. The three pairs of stimulation electrodes 84 are distributed circumferentially to provide circumferential coverage of nerves. Each pair of stimulating electrodes 84 can be energized to evoke responses to be recorded with the recording electrodes 86.

FIG. 5 shows different configurations of the stimulating and recording electrodes of the deployable sleeve 80 of FIG. 4. The elevational view of FIG. 5A and the end view of FIG. 5B (from proximal end) illustrate the arrangement of the three pairs of stimulating electrodes 84 and three recording electrodes 86. The ablation electrodes are omitted for simplicity. FIG. 5C shows a recording configuration with the first pair of stimulation electrodes 84 active (other pairs are omitted for simplicity). The active stimulation electrodes 84 and recording electrodes 86 are connected to recording amplifiers 88. FIG. 5D shows a recording configuration with the second pair of stimulation electrodes 84 active (other pairs are omitted for simplicity). FIG. 5E shows a recording configuration with the third pair of stimulation electrodes 84 active (other pairs are omitted for simplicity). FIG. 5F shows a recording configuration with all three pairs of stimulation electrodes 84 active. A total of three contiguous stimulation-recording periods can be performed, for both baseline and post-denervation assessments. Alternatively, all three stimulating electrode pairs 86 are energized simultaneously, while evoked responses are recorded between each recording electrode 86 and an indifferent electrode or ground.

Lead wires for all the electrodes as well as thermocouple wires are embedded within the sleeve substrate. During ablation the ablation electrode circuit is closed, while the neural assessment electrode circuit is open. Following ablation, the opposite is true for neural assessment, i.e., the ablation electrode circuit is open, and the neural assessment electrode circuit is closed. Additional electromechanical sensors for force/pressure sensing and the like can likewise be incorporated as the sensors 66 on the balloons 60, 70 of FIGS. 2 and 3. The sleeve 80 may be made of a variety of materials including, for example, polyurethane or nylon.

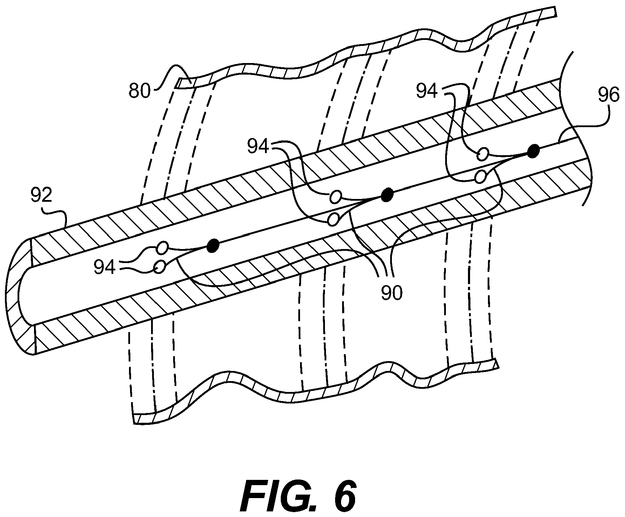

FIG. 6 is a perspective view of a partially cut-out section of the catheter device of FIG. 4. The sleeve 80 is moved between a deployed arrangement and a contracted/undeployed arrangement using any suitable mechanism. The sleeve 80 is open and non-occlusive, except for the mechanism of deploying and contracting. In the example seen in FIG. 6, the sleeve 80 is contracted using three sets of draw strings 90 located near the distal and proximal ends as well as midpoint of the sleeve 80. The draw strings 90 are drawn into a hollow tubing 92, which is capped at the distal end, through the holes 94 per draw string 90. All three draw strings 90 are joined together at a proximal distance by a pull wire 96 that is controlled at the proximal catheter handle 46. To release the draw strings 90, the handle 46 will first advance the pull wire 96 to relax the draw strings 90, and then a bolus of saline or the like is injected through the tubing 92 creating an outward pressure at the draw string holes 94 (and optionally additional holes) and forces the sleeve 80 to expand towards the vascular wall, thus insulating the electrodes from the vessel. To move the sleeve 80 to the contracted arrangement, the pull wire 96 is retracted at the handle 46 to pull the draw strings 90 to draw the sleeve 80 radially inwardly. The non-occlusive sleeve 80 is deployed by exerting a positive differential pressure by the injected fluid against the blood in the vessel, and once deployed, is kept in place (i.e., in contact with vessel wall) by the blood pressure. In the reverse action of contracting, the draw strings 90 are retracted into the hollow tubing 92 by the pull wire 96, having overcome the positive blood pressure exerted against the sleeve 80.

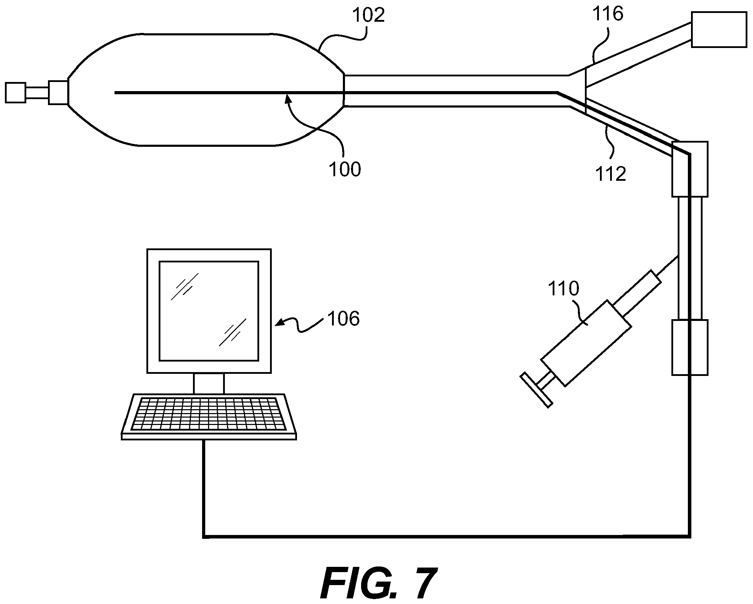

FIG. 7 is a schematic view illustrating an apparatus to provide an intraluminal pressure sensor for use with the catheter device. In the embodiment shown, the intraluminal pressure sensor 100 is introduced proximally and deployed inside the balloon 102 (which may be balloon 60 of FIG. 2 or balloon 70 of FIG. 3) for pressure monitoring while maintaining the balloon 102 isovolumic. The apparatus of FIG. 7 includes an analyzer 106 coupled to the pressure sensor 100, a syringe 110 and a fluid port 112 for introducing a fluid into the balloon 102, and an electrical port 116 for supplying electrical energy to the catheter device. This provides an alternative or an additional mechanism for pressure sensing while maintaining the balloon 102 isovolumic.

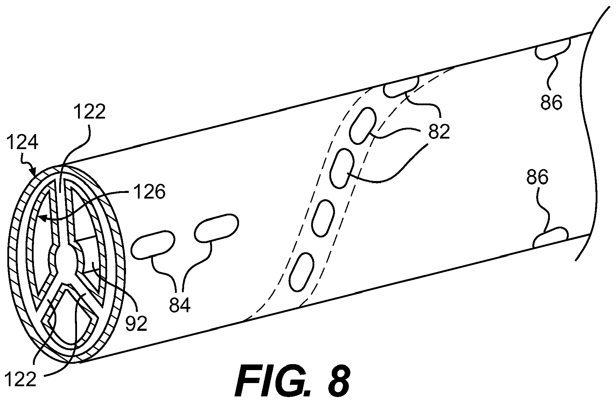

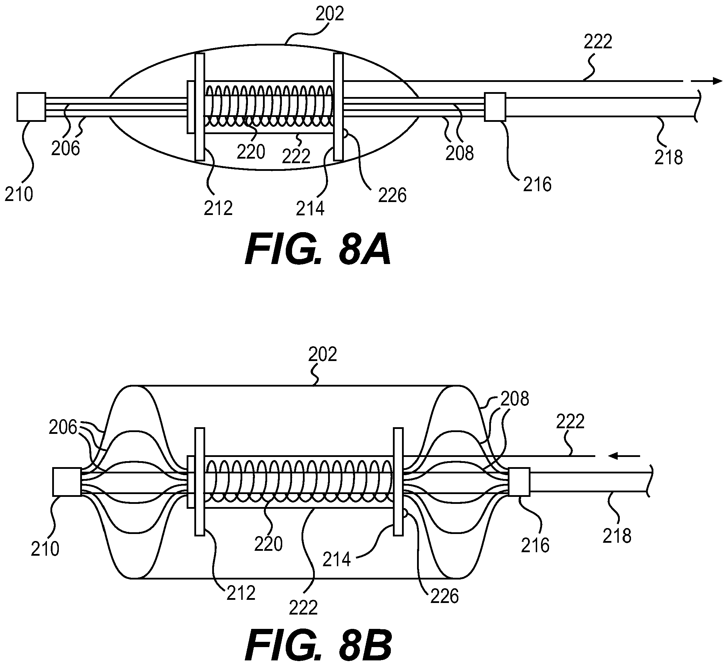

FIG. 8 is a perspective view of a catheter device with a deployable sleeve having an anti-occlusion feature according to another embodiment of the present invention. This embodiment is similar to that of FIGS. 4 and 5 but the sleeve structure is modified to provide an anti-occlusion feature to permit blood/fluid flow in the vessel between the proximal end and the distal end of the deployable sleeve structure in the deployed arrangement. In FIG. 8, the sleeve 120 remains flexible and has electrodes similar to those in FIG. 4. A plurality of rib channels 122 are used to connect the lumen of the hollow tubing 92 to an enclosed interior of sleeve 120 between its outer shell 124 and inner shell 126. The interior of the sleeve 120 and the rib channels 122 are capped or closed at the proximal and distal ends. The inner shell 126 of the sleeve 120 is preferably noncompliant so that it does not stretch or expand beyond its preset expanded shape. The outer shell 124 of the sleeve 120 is preferably semi-compliant so that it can stretch under the pressure of the fluid supplied to the interior of the sleeve 120 to enhance contact between the electrodes and the vessel wall. The rib channels 122 span the length of the sleeve 120 and are preferably flexible but noncompliant. Open flow paths are provided between the rib channels 122 to permit fluid flow in the vessel between the proximal end and the distal end of the sleeve 120 in the deployed arrangement.

FIGS. 8A and 8B are elevational views of a catheter device with a deployable sleeve having an anti-occlusion feature according to another embodiment of the present invention. The sleeve 202 is undeployed in FIG. 8A and deployed in FIG. 8B. The sleeve 202 is connected or bonded to a set of distal spines 206 and a set of proximal spines 208. The distal spines 206 are connected between a distal termination end 210 and a distal retainer 212. The proximal spines 208 are connected between a proximal retainer 214 and a proximal termination end 216. The distal termination end 210 and proximal termination end 216 are both fixed to the catheter body 218, and are hence separated by a fixed distance. A resilient member such as a spring 220 is connected between the distal retainer 212 and the proximal retainer 214. A wire 222, which is a pull wire or a push-pull wire, is fixed at one end to a wire anchor 226 on the proximal retainer 214 and loops through holes in the proximal retainer 214 and the distal retainer 212 to the proximal portion of the catheter body 218. In FIG. 8A, the wire 222 is pulled to produce a precompressed spring 220. The distal spines 206 and proximal spines 208 as well as the sleeve 202 are in the collapsed/contracted/undeployed state. FIG. 8B shows the distal spines 206, proximal spines 208, and sleeve 202 in the expanded/deployed state. By releasing the tension on the wire 222, the spring 220 stretches, thereby pushing the distal retainer 212 and proximal retainer 214 apart. The distal retainer 212 moves toward the distal termination end 210 and the proximal retainer 214 moves toward the proximal termination end 216. This causes the distal spines 206 and the proximal spines 208 to expand laterally and deploy the sleeve 202 radially outwardly. The ends of the sleeve 202 are open in the deployed state of FIG. 8B, thereby allowing fluid flow in the vessel between the proximal end and the distal end of the sleeve 202 in the deployed arrangement without occlusion.

Other configurations of deployable structures and features can be used. For example, a deployable structure may be formed by a plurality of longitudinal strips that can be pulled in the longitudinal direction toward a straight configuration in the contracted/undeployed arrangement. When the pulling force is removed, the longitudinal strips expand radially outwardly into a spiral configuration in the deployed arrangement, for instance, under a resilient biasing force such as a memory shape material. Another example of a deployable structure is an S-shaped structure having one or more stimulating elements at the distal end thereof, one or more ablation elements on the first/distal hump of the S-shaped structure from the distal end thereof, and one or more recording elements on the second/proximal hump of the S-shaped structure from the distal end thereof. The S-shaped structure is preformed into the S-shape (e.g., using a shape memory material). It is stretched/deformed toward a straight configuration (e.g., using a stylet) in the contracted arrangement and is allowed to return to the S-shape in the deployed arrangement.

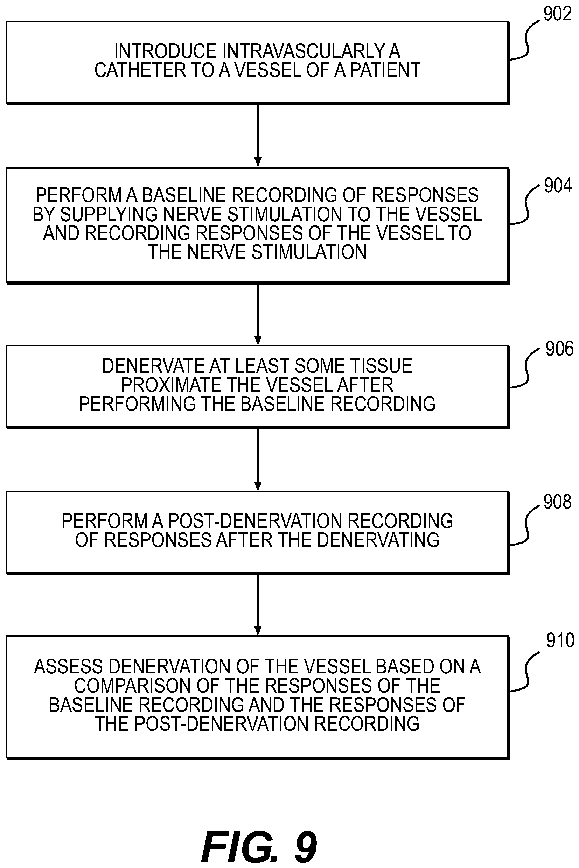

FIG. 9 shows an example of a flow diagram illustrating a method for assessing transvascular denervation. To assess transvascular denervation using the apparatus described above, a user introduces intravascularly any of the above catheter devices to a target vessel of a patient and deploys it (step 902), and performs a baseline recording of responses by supplying nerve stimulating signals to the vessel with the stimulation electrodes and records responses of the vessel to the nerve stimulating signals using the recording electrodes (step 904). Next, the ablation electrodes are activated to ablate/denervate at least some tissue proximate the vessel (step 906). The user then performs a post-ablation recording of responses, after the denervating, by supplying nerve stimulating signals to the vessel with the stimulation electrodes and records responses of the vessel to the nerve stimulating signals using the recording electrodes (step 908). The user can assess denervation of the vessel based on a comparison of the responses of the baseline recording and the responses of the post-ablation recording (step 910). For stimulation and recording, it is preferable to deploy or expand a deployable structure (e.g., basket, balloon, sleeve, or the like) to enhance contact between the stimulation electrodes and the vessel wall and between the recording electrodes and the vessel wall. Ablation/denervation elements are preferably provided on the catheter device. As such, it is not necessary to reposition the catheter during the baseline recording, the ablation, and the post-ablation recording.

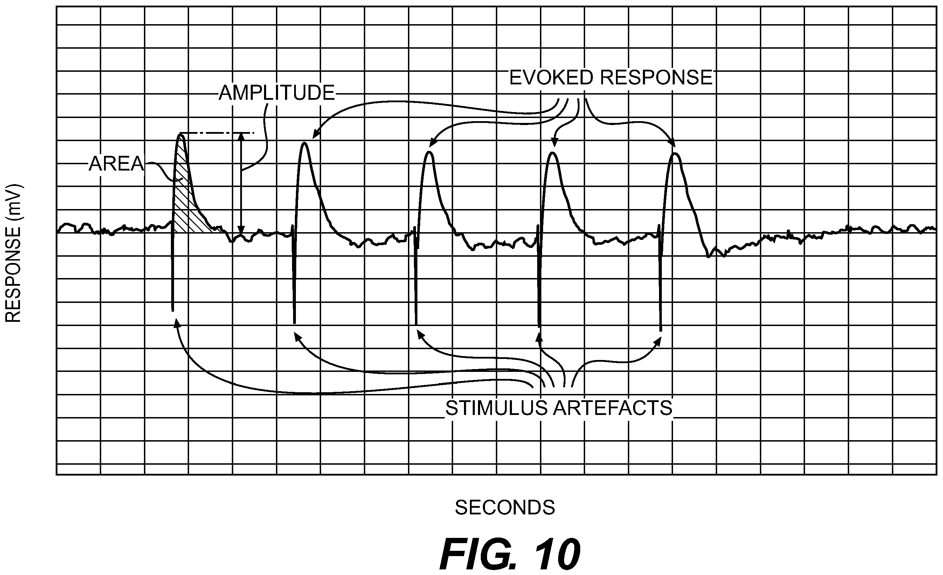

The responses include, for example, electrical response and/or mechanical response, which can be evoked electrical potentials or wall tension of the vessel in response to the nerve stimulating signals. In general, assessing denervation of the vessel includes computing a baseline parameter from the responses of the baseline recording, computing a post-ablation parameter from the responses of the post-ablation recording, and computing a degree of denervation as a ratio of the post-ablation parameter and the baseline parameter. The desired denervation is achieved when the computed ratio falls within a preset range. In one example, the baseline parameter is computed based on a baseline maximum signal amplitude of one or more evoked responses generated in response to stimulation of nerve stimulating signals by the stimulation elements and recorded by the recording elements before the ablating, and the post-ablation is computed based on a post-ablation maximum signal amplitude of one or more evoked responses generated in response to the same stimulation of nerve stimulating signals by the stimulation elements and recorded by the recording elements after the ablating. The evoked responses may include evoked electrical response and/or evoked mechanical response such as can be detected with a pressure sensor. In another example, the baseline parameter is computed based on a baseline area under a plot of one or more evoked responses generated in response to stimulation by the stimulation elements and recorded by the recording elements before the ablating, and the post-ablation is computed based on a post-ablation area under a plot of one or more evoked responses generated in response to the same stimulation by the stimulation elements and recorded by the recording elements after the ablating. FIG. 10 shows an example of a plot of evoked responses and stimulus artifacts recorded by the recording elements. The evoked responses each have an amplitude and an area. For multiple evoked responses, an average of the amplitudes or an average of the areas may be used to calculate the parameter.

If denervation of the vessel is not achieved, the user can repeat the steps of denervating (step 906), performing a post-ablation recording of responses (step 908), and assessing denervation of the vessel (step 910) until denervation of the vessel is achieved. The catheter need not be repositioned during the repeating. In some cases, repeating those steps include adjusting an energy level of ablation or a number of ablation elements for ablating tissue proximate the vessel based on the result of assessing denervation of the vessel. The repeating with or without the adjusting until denervation of the vessel is achieved is preferably performed in real time. The recording and assessing are preferably done in real time so that the assessment results can be provided as feedback to the user who can repeat the steps in real time, including adjusting the denervation in real time if necessary, to achieve the desired denervation for the patient undergoing the medical procedure in real time. This can be done, for example, by interleaving ablation cycles with neural assessment cycles throughout the therapeutic procedure. If it is desired to adjust the position of the ablation or denervation elements the step of performing a baseline evoked response measurement should be repeated after the elements are repositioned and before the subsequent denervation step.

As an example, the baseline recording of evoked responses is performed to compute a parameters Ai which can be derived from a number of variables such as the maximum signal amplitude or area under the first volley as mentioned above. The post-ablation assessment yields a second parameter Ao. The degree of denervation is computed as the DNAi given by Ao/Ai. The neural assessment method can be extended to provide closed loop control of denervation by interleaving ablation with neural assessment, for example, by incrementing the number of electrodes involved in ablation after each neural assessment until the DNAi is reduced below a certain threshold or falls within a preset range. This approach minimizes the need to move the catheter to different segments of the vessel for total coverage of denervation. Neural assessments can be done with bipolar or unipolar stimulation, but preferably bipolar. The bipolar stimulus can be either constant current or constant voltage, and is preferably a square wave with a pulse duration of about 0.1-1 millisecond, more preferably about 0.2-0.5 millisecond, and an amplitude of about 1-20 mA or 1-20 V, more preferably about 5-10 mA or 5-10 V. The stimulus is repeated typically at a rate of about 0.5-20 Hz for a total of about 5-30 seconds.

An alternative to electrical stimulation is pharmacological stimulation. Examples include the use of alpha-latrotoxin or ciguatoxin, which can be applied extraluminally (e.g., at 1 nM dose) to the renal artery. The drug can be delivered using a micro-infusion needle that penetrates the arterial wall to reach the perivascular axons. For instance, a balloon can be used to drive the needle into the arterial wall. The drug can temporarily activate or accelerate the release of neurotransmitters from perivascular varicosities and thus generate the evoked response for signal recording. The micro-infusion needle will replace the stimulation electrodes of the above embodiments while the recording electrodes are still used to record the response to the nerve stimulation produced by the drug.

The above-described method provides a direct and immediate assessment of the transvascular denervation procedure. It ensures optimal titration of energy to achieve denervation end point and provides a way to predict clinical outcome of denervation, preferably in real time.

In the description, numerous details are set forth for purposes of explanation in order to provide a thorough understanding of the present invention. However, it will be apparent to one skilled in the art that not all of these specific details are required in order to practice the present invention.

From the foregoing, it will be apparent that the invention provides apparatuses for ablation using an irrigated catheter device with multiple segmented ablation segments. Additionally, while specific embodiments have been illustrated and described in this specification, those of ordinary skill in the art appreciate that any arrangement that is calculated to achieve the same purpose may be substituted for the specific embodiments disclosed. This disclosure is intended to cover any and all adaptations or variations of the present which fall under the scope of the claims, and it is to be understood that the terms used in the following claims should not be construed to limit the invention to the specific embodiments disclosed in the specification. Rather, the scope of the invention is to be determined entirely by the following claims.

## Claims

1. A catheter apparatus for assessing denervation comprising:
an elongated catheter body (40) having a proximal end (42) and a distal end (44), a longitudinal axis extending in a longitudinal direction between the distal end (44) and the proximal end (42);
a deployable structure coupled to the catheter body (40), the deployable structure being deployable outwardly from the longitudinal axis of the catheter_ body (40) to a deployed arrangement and contractible inwardly toward the longitudinal axis of the catheter body (40) to a contracted arrangement, the deployable structure comprising a deployable sleeve (80), wherein at least a portion of the catheter body (40) is disposed inside the deployable sleeve (80), and an anti-occlusion feature configured to permit fluid flow in a vessel of a patient between a proximal end and a distal end of the deployable structure in the deployed arrangement;
one or more ablation elements (82) disposed on the deployable sleeve (80) to move outwardly and inwardly with the deployable sleeve (80), the one or more ablation elements (82) suitable for being powered to apply ablation energy to the vessel;
one or more stimulation elements (84) spaced from each other and disposed on the deployable sleeve (80) to move outwardly and inwardly with the deployable sleeve (80), the one or more stimulation elements (84) being powered to supply nerve stimulating signals to the vessel; and
one or more recording elements (86) spaced from each other and from the one or more stimulation elements (84), the one or more recording elements (86) being disposed on the deployable sleeve (80) to move outwardly and inwardly with the deployable sleeve (80) and being configured to record evoked electrical responses of the vessel in response to the nerve stimulating signals

2. The catheter ablation apparatus of claim 1,
wherein the one or more stimulation elements (84) are proximal relative to the one or more recording elements (86).

3. The catheter apparatus of claim 1,
wherein a most distal ablation element of the one or more ablation elements (82) is no more distal than at least one of the stimulation elements (84) and a most proximal ablation element of the one or more ablation elements (82) is no more proximal than at least one of the one or more recording elements (86).

4. The catheter apparatus of claim 1,
wherein at least one of the ablation elements (82) is also a stimulation element (84) or a recording element (86).

5. The catheter apparatus of claim 1,
wherein some of the one or more recording elements (86) are spaced from one of the one or more stimulation elements (84) by one of a longitudinal spacing, a lateral spacing, or a combined longitudinal and lateral spacing.

6. The catheter apparatus of claim 1,
wherein the one or more recording elements (86) are configured to record evoked electrical responses of the vessel in response to the nerve stimulating signals by direct contact with a vessel luminal wall of the vessel.

7. The catheter apparatus of claim 1,
wherein the portion of the catheter body (40) disposed inside the deployable sleeve (80) includes a plurality of holes for fluid to pass therethrough to facilitate pushing the deployable sleeve (80) radially outwardly relative to the catheter body (40) in a deployed arrangement.

8. The catheter apparatus of claim 7, further comprising
a plurality of draw strings (90) extending from inside the portion of the catheter body (40) disposed inside the deployable sleeve (80) through the plurality of holes to the deployable sleeve (80) to draw the deployable sleeve (80) radially inwardly relative to the catheter body (40) in an undeployed arrangement.

9. The catheter apparatus of claim 1,
wherein the deployable sleeve (80) comprises an electrically insulative material.

10. The catheter apparatus of claim 1 further comprising:
a mechanism to deploy the structure outwardly from the longitudinal axis of the catheter body (40) to move the ablation elements (82), the one or more stimulation elements (84), and the one or more recording elements (86) outwardly to the deployed arrangement, and to contract the structure inwardly toward the longitudinal axis of the catheter body (40) to move the ablation elements (82), the one or more stimulation elements (84), and the one or more recording elements (86) inwardly to the contracted arrangement

## Patentansprüche

1. Kathetervorrichtung zur Beurteilung von Denervierung umfassend:
einen länglichen Katheterkörper (40) mit einem proximalen Ende (42) und einem distalen Ende (44), eine Längsachse, die sich in einer Längsrichtung erstreckt zwischen dem distalen Ende (44) und dem proximalen Ende (42);
eine entfaltbare Struktur, die mit dem Katheterkörper (40) gekoppelt ist, wobei die entfaltbare Struktur nach außen von der Längsachse des Katheterkörpers (40) in eine entfaltete Anordnung entfaltbar ist und nach innen, in Richtung der Längsachse des Katheterkörpers (40), zu einer zusammengezogenen Anordnung zusammenziehbar ist, wobei die entfaltbare Struktur einen entfaltbaren Schlauch (80) umfasst, wobei zumindest ein Abschnitt des Katheterkörpers (40) innerhalb des entfaltbaren Schlauchs (80) angeordnet ist, und eine Anti-Verschluss-Eigenschaft, welche konfiguriert ist, um Fluiddurchfluss zwischen einem proximalen Ende und einem distalen Ende der entfaltbaren Struktur in der entfalteten Anordnung in einem Gefäß eines Patienten zu erlauben;
ein oder mehrere Ablationselemente (82), die auf dem entfaltbaren Schlauch (80) angeordnet sind, um sich nach außen und innen mit dem entfaltbaren Schlauch (80) zu bewegen, wobei das eine oder mehrere Ablationselemente (82) zur Versorgung mit Strom geeignet sind, um dem Gefäß Ablationsenergie zuzuführen;
ein oder mehrere Stimulationselemente (84), die im Abstand voneinander gesetzt und auf dem entfaltbaren Schlauch (80) angeordnet sind, um sich nach außen und innen mit dem entfaltbaren Schlauch (80) zu bewegen, wobei das eine oder mehrere Stimulationselemente (84) mit Strom versorgt werden, um nervenstimulierende Signale dem Gefäß zuzuführen; und
ein oder mehrere Aufzeichnungselemente (86), die im Abstand voneinander und von dem einen oder mehreren Stimulationselementen (84) gesetzt sind, wobei das eine oder mehrere Aufzeichnungselemente (86) auf dem entfaltbaren Schlauch (80) angeordnet sind, um sich nach außen und innen mit dem entfaltbaren Schlauch (80) zu bewegen und konfiguriert sind, um evozierte elektrische Antworten des Gefäßes in Antwort auf die nervenstimulierenden Signale aufzuzeichnen.

2. Katheterablationsvorrichtung nach Anspruch 1,
wobei das eine oder mehrere Stimulationselemente (84) proximal in Verhältnis zu dem einen oder mehreren Aufzeichnungselementen (86) sind.

3. Kathetervorrichtung nach Anspruch 1,
wobei ein am weitesten distales Ablationselement von dem einen oder mehreren Ablationselementen (82) nicht weiter distal ist, als zumindest eines von den Stimulationselementen (84) und ein am nächsten proximales Ablationselement dem einen oder mehreren Ablationselementen (82) nicht näher proximal ist, als zumindest eines von dem einem oder mehreren Aufzeichnungselementen (86).

4. Kathetervorrichtung nach Anspruch 1,
wobei zumindest eines von den Ablationselementen (82) ebenfalls ein Stimulationselement (84) oder ein Aufzeichnungselement (86) ist.

5. Kathetervorrichtung nach Anspruch 1,
wobei manche von dem einen oder mehreren Aufzeichnungselementen (86) von einem oder mehreren Stimulationselementen (84) in einem Abstand in Längsrichtung, einem seitlichen Abstand oder einem kombinierten Abstand in Längsrichtung und seitlich entfernt sind.

6. Kathetervorrichtung nach Anspruch 1,
wobei eines oder mehrere der Aufzeichnungselemente (86) konfiguriert sind, um evozierte elektrische Antworten des Gefäßes in Antwort auf die nervenstimulierenden Signale, durch direkten Kontakt mit einer Gefäßluminalwand des Gefäßes aufzuzeichnen.

7. Kathetervorrichtung nach Anspruch 1,
wobei ein Abschnitt des Katheterkörpers (40), der innerhalb des entfaltbaren Schlauchs (80) angebracht ist, eine Vielzahl von Öffnungen zum Durchtritt von Fluid dort hindurch enthält, um Schieben des entfaltbaren Schlauches (80) radial nach außen im Verhältnis zu dem Katheterkörper (40) in einer entfalteten Anordnung zu erleichtern.

8. Kathetervorrichtung nach Anspruch 7, ferner umfassend
eine Vielzahl von Zugleinen (90), die sich vom Inneren des Abschnitts des Katheterkörpers (40), der innerhalb des entfaltbaren Schlauches (80) angebracht ist, durch eine Vielzahl von Öffnungen bis zu dem entfaltbaren Schlauch (80) erstrecken, um den entfaltbaren Schlauch (80) radial nach innen in Verhältnis zu dem Katheterkörper (40) in einer nicht-entfalteten Anordnung zu ziehen.

9. Kathetervorrichtung nach Anspruch 1,
wobei der entfaltbare Schlauch (80) ein elektrisch isolierendes Material umfasst.

10. Kathetervorrichtung nach Anspruch 1, ferner umfassend:
einen Mechanismus zur Entfaltung der Struktur von der Längsachse des Katheterkörpers (40) nach außen, um die Ablationselemente (82), das eine oder mehrere Stimulationselemente (84) und das eine oder mehrere Aufzeichnungselemente (86) nach außen, zu der entfalteten Anordnung zu bewegen und um die Struktur nach innen, zu der Längsachse des Katheterkörpers (40) zusammenzuziehen, um die Ablationselemente (82), das eine oder mehrere Stimulationselemente (84) und das eine oder mehrere Aufzeichnungselemente (86) nach innen, in die zusammengezogene Anordnung zu bewegen.

## Revendications

1. Appareil de cathéter pour évaluer une dénervation comprenant :
un corps de cathéter allongé (40) ayant une extrémité proximale (42) et une extrémité distale (44), un axe longitudinal s'étendant dans une direction longitudinale entre l'extrémité distale (44) et l'extrémité proximale (42) ;
une structure déployable couplée au corps de cathéter (40), la structure déployable pouvant se déployer vers l'extérieur depuis l'axe longitudinal du corps de cathéter (40) jusqu'à un agencement déployé et se contracter vers l'intérieur vers l'axe longitudinal du corps de cathéter (40) jusqu'à un agencement contracté, la structure déployable comprenant un manchon déployable (80), dans lequel au moins une partie du corps de cathéter (40) est disposée à l'intérieur du manchon déployable (80), et un élément anti-occlusion configuré pour permettre un écoulement de fluide dans un vaisseau d'un patient entre une extrémité proximale et une extrémité distale de la structure déployable dans l'agencement déployé ;
un ou plusieurs éléments d'ablation (82) disposés sur le manchon déployable (80) pour se déplacer vers l'extérieur et vers l'intérieur avec le manchon déployable (80), le ou les éléments d'ablation (82) étant appropriés pour être alimentés afin d'appliquer de l'énergie d'ablation au vaisseau ;
un ou plusieurs éléments de stimulation (84) espacés les uns des autres et disposés sur le manchon déployable (80) pour se déplacer vers l'extérieur et vers l'intérieur avec le manchon déployable (80), le ou les éléments de stimulation (84) étant alimentés pour fournir des signaux de stimulation nerveuse au vaisseau ; et
un ou plusieurs éléments d'enregistrement (86) espacés les uns des autres et du ou des éléments de stimulation (84), le ou les éléments d'enregistrement (86) étant disposés sur le manchon déployable (80) pour se déplacer vers l'extérieur et vers l'intérieur avec le manchon déployable (80) et étant configurés pour enregistrer des réponses électriques évoquées du vaisseau en réaction aux signaux de stimulation nerveuse.

2. Appareil d'ablation à cathéter selon la revendication 1,
dans lequel le ou les éléments de stimulation (84) sont proximaux par rapport à un ou plusieurs éléments d'enregistrement (86).

3. Appareil de cathéter selon la revendication 1,
dans lequel un élément d'ablation le plus distal du ou des éléments d'ablation (82) n'est pas plus distal qu'au moins un des éléments de stimulation (84) et un élément d'ablation le plus proximal du ou des éléments d'ablation (82) n'est pas plus proximal qu'au moins un du ou des éléments d'enregistrement (86).

4. Appareil de cathéter selon la revendication 1,
dans lequel au moins un des éléments d'ablation (82) est également un élément de stimulation (84) ou un élément d'enregistrement (86).

5. Appareil de cathéter selon la revendication 1,
dans lequel certains du ou des éléments d'enregistrement (86) sont espacés de l'un du ou des éléments de stimulation (84) de l'un parmi un espacement longitudinal, un espacement latéral, ou un espacement longitudinal et latéral combiné.

6. Appareil de cathéter selon la revendication 1,
dans lequel le ou les éléments d'enregistrement (86) sont configurés pour enregistrer des réponses électriques évoquées du vaisseau en réponse aux signaux de stimulation nerveuse par contact direct avec une paroi luminale de vaisseau du vaisseau.

7. Appareil de cathéter selon la revendication 1,
dans lequel la partie du corps de cathéter (40) disposée à l'intérieur du manchon déployable (80) inclut une pluralité de trous pour que le fluide y passe à travers afin de faciliter la poussée du manchon déployable (80) radialement vers l'extérieur par rapport au corps de cathéter (40) dans un agencement déployé.

8. Appareil de cathéter selon la revendication 7, comprenant en outre une pluralité de cordons de traction (90) s'étendant depuis l'intérieur de la partie du corps de cathéter (40) disposée à l'intérieur du manchon déployable (80) à travers la pluralité de trous jusqu'au manchon déployable (80) afin de tirer le manchon déployable (80) radialement vers l'intérieur par rapport au corps de cathéter (40) dans un agencement non déployé.

9. Appareil de cathéter selon la revendication 1, comprenant en outre
dans lequel le dispositif déployable (80) comprend un matériau électriquement isolant.

10. Appareil de cathéter selon la revendication 1 comprenant en outre :
un mécanisme pour déployer la structure vers l'extérieur depuis l'axe longitudinal du corps de cathéter (40) afin de déplacer les éléments d'ablation (82), le ou les éléments de stimulation (84), et le ou les éléments d'enregistrement (86) vers l'extérieur jusqu'à l'agencement déployé, et pour contracter la structure vers l'intérieur vers l'axe longitudinal du corps de cathéter (40) afin de déplacer les éléments d'ablation (82), le ou les éléments de stimulation (84) et le ou les éléments d'enregistrement (86) vers l'intérieur jusqu'à l'agencement contracté.
